Europäisches Patentamt

(19) European Patent Office

Office européen des brevets

(11) Numéro de publication : **0 299 002 B1**

(12) **FASCICULE DE BREVET EUROPEEN**

(45) Date de publication du fascicule du brevet :
07.08.91 Bulletin 91/32

(21) Numéro de dépôt : **88900385.1**

(22) Date de dépôt : **18.12.87**

(86) Numéro de dépôt international :
**PCT/FR87/00508**

(87) Numéro de publication internationale :
**WO 88/04914 14.07.88 Gazette 88/15**

(51) Int. Cl.⁵ : **A61F 2/02, A61F 2/12**

(54) **PROTHESE A EXPANSION TISSULAIRE.**

(30) Priorité : 26.12.86 FR 8618474

(43) Date de publication de la demande :
18.01.89 Bulletin 89/03

(45) Mention de la délivrance du brevet :
07.08.91 Bulletin 91/32

(84) Etats contractants désignés :
BE DE FR GB IT

(56) Documents cités :
EP-A-01 817 20
US-A- 4 217 889

(73) Titulaire : **Mai, Christian**
**74 boulevard des Belges**
**F-69006 Lyon (FR)**
Titulaire : **CRASSAS, Yves**
**Clinique Saint-Charles Rue Fernand-Léger**
**F-38150 Roussillon (FR)**

(72) Inventeur : **Mai, Christian**
**74 boulevard des Belges**
**F-69006 Lyon (FR)**
Inventeur : **CRASSAS, Yves**
**Clinique Saint-Charles Rue Fernand-Léger**
**F-38150 Roussillon (FR)**

(74) Mandataire : **Laurent, Michel**
**Cabinet LAURENT et CHARRAS, 20, rue Louis**
**Chirpaz B.P. 32**
**F-69131 Ecully Cedex (FR)**

## Description

L'invention concerne un nouveau type de prothèse à expansion tissulaire.

Par "prothèse à expansion tissulaire", on désigne des prothèses dans lesquelles on gonfle un corps souple approprié pour dilater les tissus et/ou la peau pour leur donner des formes appropriées. Ce type de prothèse gonflable qui correspond au préambule de la revendication 1 est bien connu en chirurgie plastique (voir par exemple EP-A-0181720, US-A-4217889).

Les prothèses de ce type actuellement disponibles sont généralement volumineuses car leur forme, leur volume et leur surface initiale correspondent sensiblement à la surface des tissus ou de peau à dilater. De la sorte, l'implantation de ces prothèses nécessite de pratiquer une large incision et une voie (ouverture) d'abords avec un décollement appréciable. La mise en place de ces prothèses nécessite une technique complexe effectuée à ciel ouvert. Cela implique un temps opératoire et d'exposition relativement long avec donc des risques septiques non négligeables. Le gonflage de la prothèse compte-tenu de l'importance de l'incision ne peut guère être effectuée avant le douzième jour et souvent même plus tard et ce, en fonction de la rapidité de la cicatrisation de la voie d'abords. Fréquemment, des complications provoquées par des désunions, voire par des nécroses cutanées, nécessitent parfois l'interruption momentanée ou définitive du processus de reconstruction.

L'invention pallie ces inconvénients. Elle concerne une prothèse à expansion tissulaire qui soit peu volumineuse et dans laquelle l'expansion par gonflage peut être dirigée et contrôlée.

La prothèse selon l'invention concerne également une réalisation qui nécessite seulement deux faibles incisions en amont et en aval de la zone implantée. De la sorte, les complications et les risques septiques sont fortement réduits et les risques de désunion sur cicatrices longues sont évités, de même que les risques de nécroses par ischémie tissulaire et chute d'escarres.

Ainsi, grâce à l'expansion dirigée, on évite les débordements sur la cicatrice et on obtient des lambeaux de meilleure qualité avec moins de décollements.

La prothèse à expansion tissulaire selon l'invention qui comprend un corps souple et creux fermé, en un matériau biocompatible, apte à se dilater sous l'effet d'une pression de fluide, destiné à être inséré sous le tissu et/ou la peau à réparer, qui comporte un raccord souple destiné à le relier à une source de fluide de dilatation, se caractérise en ce que l'ensemble : corps souple et raccord est inséré dans une enveloppe creuse rigide dont l'une au moins des extrémités est arrondie.

Dans une forme de réalisation préférée, le corps souple creux dilatable et l'enveloppe creuse rigide sont de formes tubulaires allongées coaxiales, de section circulaire ou autre.

En d'autres termes, l'invention se caractérise essentiellement en ce que le corps souple dilatable est tubulaire et est inséré dans une enveloppe rigide dont l'extrémité est arrondie, ce qui facilite l'introduction de l'ensemble sous la peau. Une fois en place, on retire l'enveloppe rigide en laissant sur place le corps souple tubulaire qui grâce à son raccord, pourra être le moment venu gonflée.

Avantageusement, en pratique :
— le corps souple est en silicone, matière qui présente l'avantage d'être inerte, biocompatible et facilement dilatable de plusieurs fois son volume initial, par exemple de huit à dix fois ;
— le raccord de dilatation est constitué par un tube souple, en caoutchouc de synthèse biocompatible, soudé à une extrémité du corps dilatable;
— le corps dilatable présente au moins une surépaisseur sur tout un secteur longitudinal ; de la sorte, lors de la mise en place, cette surépaisseur qui forme semelle repose sur le substrat et lorsque l'on effectue la dilatation, on réalise ainsi une expansion dirigée et contrôlée vers les parties plus minces ;
— le raccord de dilatation est relié à une valve placée également dans l'enveloppe rigide, notamment sur le prolongement de la surépaisseur formant semelle ;
—l'enveloppe rigide en matière plastique par exemple, comporte à son extrémité arrondie un cordon souple fixé d'un côté à cette extrémité arrondie et de l'autre à une tige rigide, l'ensemble étant alors protégé par un capot emmanché sur l'extrémité arrondie.

La manière dont l'invention peut être réalisée et les avantages qui en découlent ressortiront mieux de l'exemple de réalisation qui suit donné à titre indicatif et non limitatif, à l'appui des figures annexées.

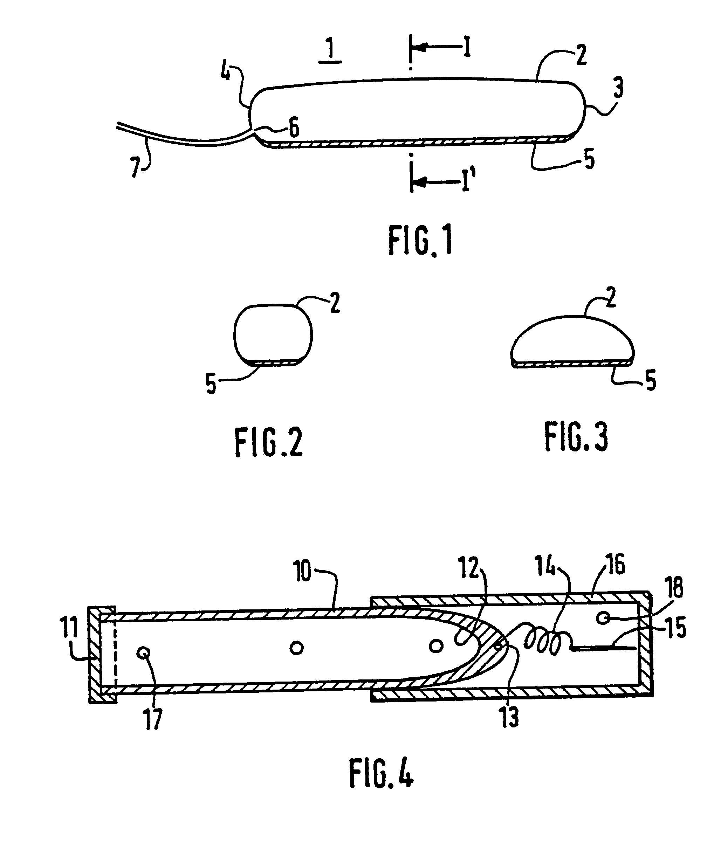

La figure 1 est une représentation simplifiée en coupe longitudinale d'un corps souple gonflable conforme à l'invention montré en section selon l'axe I-I' à la figure 2.

La figure 3 est une autre représentation en section d'une autre forme de réalisation de ce corps souple.

La figure 4 est une représentation en coupe d'une enveloppe caractéristique de l'invention.

La figure 5 représente toujours en coupe longitudinale une prothèse complète conforme à l'invention.

La figure 6 montre en coupe longitudinale une forme d'exécution perfectionnée de l'invention.

Selon l'une première caractéristique de l'invention, le corps souple gonflable destiné à être inséré sur la peau, désigné par la référence générale (1), a la forme d'un tube (2) allongé, par exemple en silicone, fermé à ses deux extrémités (3) et (4) et présentant sur tout un secteur longitudinal (5) une surépaisseur, par exemple du double de l'épaisseur moyenne du corps. De la sorte, lors du gonflage, l'expansion du corps (2) s'effectue de manière dirigée vers le haut et latéralement.

L'extrémité (4) de ce corps (2) présente une petite ouverture (6) à laquelle est soudé un raccord (7) constitué par un tube souple en caoutchouc de synthèse biocompatible.

Dans une forme de réalisation avantageuse, le corps (2) est comme déjà dit en silicone. Il a avantageusement une forme tubulaire de dix centimètres de longueur et deux centimètres de diamètre, ayant une épaisseur moyenne de 0,3 millimètre. Ce tube présente sur tout un secteur longitudinal de un centimètre et demi de large une surépaisseur (5) de un millimètre environ formant semelle.

Selon une troisième caractéristique de l'invention, ce corps tubulaire (2) et le raccord (7) sont placés dans une enveloppe rigide en matière plastique (10) également tubulaire, dont une extrémité est fermée par un couvercle (11) et dont l'autre extrémité (12) opposée à (7) a la forme d'un obus arrondi. L'extrémité (13) de cet obus présente un cordon replié (14), par exemple un fil en polyamide, à l'extrémité duquel est fixée une tige (15) formant aiguille. L'ensemble pointe-obus (13) et cordon (14) est protégé à son tour par un capot (16) emmanché sur l'extrémité arrondie (13).

L'enveloppe (10) et le capot (16) présentent des orifices (17, 18) destinés à permettre la stérilisation de l'ensemble.

Pour mettre en place le corps tubulaire dilatable (2) dans l'enveloppe (10), il suffit de retirer le capot (11), d'insérer l'ensemble (2, 7) et de refermer ce capot.

La mise en place de cette prothèse s'effectue facilement. Le chirurgien repère tout d'abord les sites de d'implantation en effectuant un dessin préalable des futurs lambeaux. Il choisit la prothèse tubulaire (2) en conséquence.

L'insertion de la prothèse s'effectue par tunnellisation sans abord direct à l'aide de deux courtes incisions en amont et en aval de la zone implantée.

Le chirurgien retire ensuite le capot (16), introduit l'aiguille (15) sous la peau à partir de l'incision en aval jusqu'à la faire ressortir par l'incision en amont, puis tire à la main sur cette aiguille (15) de manière à insérer la pointe en forme d'obus (13) dans le tunnel. Lorsque le tube rigide (10) a pénétré complètement dans la zone implantée, le chirurgien qui a dégagé le couvercle (11) retient à la main par le raccord (7), le corps tubulaire dilatable (2) et continue à exercer la traction sur le cordon souple (14) jusqu'à ressortir complètement l'enveloppe rigide (10). A ce moment, le corps dilatable (2) se trouve parfaitement en place dans la zone implantée. A ce moment, il suffit de fermer les incisions au surjet sur drain aspiratif. Grâce à une valve non représentée, branchée le moment venu au raccord (7), l'ensemble étant implanté de manière classique on assure l'expansion du corps tubulaire dilatable (2), notamment en injectant dans celui-ci du sérum physiologique.

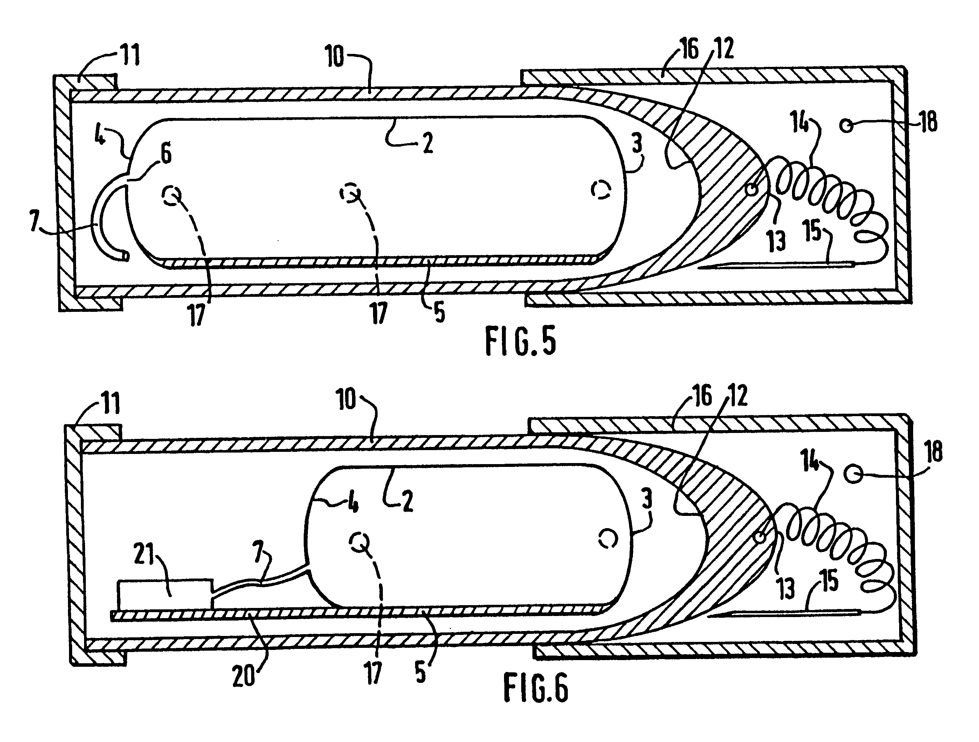

Dans une variante perfectionnée montrée à la figure 6, la surépaisseur (5) formant semelle est prolongée au delà du corps (2). Ce prolongement (20) reçoit une valve (21) reliée par le raccord (7) au corps (2). Cette disposition simplifie la mise en place.

En d'autres termes, la prothèse selon l'invention se distingue des prothèses commercialisées à ce jour, essentiellement par le fait que l'élément tubulaire dilatable est placé à l'intérieur d'un tube rigide qui joue le rôle de tunnellisateur. Ce tube est retiré après la tunnellisation, ce qui rend ainsi le corps tubulaire dilatable (2) largable.

La prothèse selon l'invention présente de nombreux avantages par rapport aux prothèses actuellement disponibles. On peut citer :

— au niveau de la mise en place, l'intervention du chirurgien devient plus simple et plus rapide et ne nécessite pas une hémostase directe ;
— la discrétion des voies d'abords permet de débuter le gonflage plus tôt que dans la technique actuelle ;
— les risques post-opératoires limités.

De la sorte, ces prothèses peuvent être utilisées avantageusement pour la réalisation de lambeaux cutanés après exérèse de cicatrices (brûlures, brides rétractiles) et pour la réalisation de détonsuration et de lambeaux du cuir chevelu.

## Revendications

1. Prothèse à expansion tissulaire, comprenant un corps souple creux fermé (2) en un matériau biocompatible, apte à se dilater sous l'effet d'une pression de fluide, destinée à être insérée sous le tissu et/ou la peau à réparer, comportant un raccord souple (7) destiné à relier le corps (2) à une source de fluide de dilatation, caractérisée en ce que l'ensemble : corps souple (2) et raccord (7) est inséré dans une enveloppe creuse rigide (10) dont l'une au moins des extrémités (12) est arrondie.

2. Prothèse à expansion tissulaire selon la revendication 1, caractérisée en ce que le corps souple creux dilatable (2) et l'enveloppe creuse rigide (10) ont une forme tubulaire allongée coaxiale.

3. Prothèse à expansion tissulaire selon la revendication 1, caractérisée en ce que le corps souple tubulaire (2) est en silicone.

4. Prothèse à expansion tissulaire selon la revendication 1, caractérisée en ce que le raccord de dilatation (7) est constitué par un tube souple en caoutchouc de synthèse biocompatible, soudé à l'extrémité (4) du corps dilatable tubulaire (2), opposé à l'extrémité arrondie (12) de l'enveloppe rigide (10).

5. Prothèse à expansion tissulaire selon la revendication 1, caractérisée en ce que le corps tubulaire dilatable (2) présente une surépaisseur (5) formant semelle sur tout un secteur longitudinal.

6. Prothèse à expansion tissulaire selon l'une des revendications 1 à 5, caractérisée en ce que l'extrémité arrondie (12) de l'enveloppe rigide (10) comporte un cordon souple (14) fixé d'un côté à cette extrémité (13) arrondie et de l'autre à une tige rigide (15), l'ensemble étant protégé à son tour par un capot (16) emmanché sur l'extrémité arrondie (12).

7. Prothèse à expansion tissulaire selon la revendication 5, caractérisée en ce que la surépaisseur (5) formant semelle est prolongée sur une extrémité (4) du corps creux (2), ce prolongement (20) recevant une valve (21) reliée par le raccord (7) au corps creux (2) dilatable.

## Patentansprüche

1. Prothese zur Expansion des Hautgewebes bestehend aus einem geschlossenen Hohlweichkörper (2) aus einem biokompatiblen Material, die sich unter Einwirkung eines Flüssigkeitsdrucks ausdehnen kann und zum Einsetzen unter das Gewebe und/oder die zu behandelnde Haut bestimmt, ausgestattet mit einem flexiblen Anschlußstück (7), das den Prothesekörper (2) mit einer Quelle für die Dehnflüssigkeit verbindet, dadurch gekennzeichnet, daß die Baueinheit aus Weichkörper (2) und Anschlußstück (7) in eine steife Hohlumwandung (10) eingefügt ist, die zumindest ein abgerundetes Ende (12) aufweist.

2. Prothese zur Expansion des Hautgewebes nach dem Anspruch 1, dadurch gekennzeichnet, daß der dehnbare Hohlweichkörper (2) und die steife Hohlumwandung (10) eine koaxiale längliche Röhrenform aufweisen.

3. Prothese zur Expansion des Hautgewebes nach dem Anspruch 1, dadurch gekennzeichnet, daß der röhrenförmige Weichkörper (2) aus Silikon besteht.

4. Prothese zur Expansion des Hautgewebes nach dem Anspruch 1, dadurch gekennzeichnet, daß der Dehnungsanschluß (7) aus einem flexiblen Rohr aus biokompatiblem Synthesegummi besteht, das am Ende (4) des dehnbaren röhrenförmigen Körpers (2) gegenüber dem abgerundeten Ende (12) der steifen Umwandung (10) verschweißt ist.

5. Prothese zur Expansion des Hautgewebes nach dem Anspruch 1, dadurch gekennzeichnet, daß der dehnbare röhrenförmige Körper (2) eine Überdicke (5) aufweist, die über einen ganzen Längsabschnitt hinweg eine Sohle bildet.

6. Prothese zur Expansion des Hautgewebes nach einem der Ansprüche 1-5, dadurch gekennzeichnet, daß das abgerundete Ende (12) der steifen Umwandung (10) eine flexible Schnur (14) besitzt, die auf einer Seite an.dem angerundeten Ende (13) und auf der anderen an einem starren Stift (15) befestigt ist, wobei das Ganze durch eine Abedeckung (16) geschützt ist, die auf das abgerundete Ende (12) aufgesetzt wird.

7. Prothese zur Expansion des Hautgewebes nach dem Anspruch 5, dadurch gekennzeichnet, daß die sohlenbildende Überdicke (5) an einem Ende (4) des Hohlkörpers (2) verlängert ist, wobei das Verlängerungsstück (20) ein Ventil (21) aufnimmt, das über den Anschluß (7) mit dem dehnbaren Hohlkörper (2) in Verbindung steht.

## Claims

1. A tissue expansion prosthesis consisting of a closed hollow flexible body (2) made of a biocompatible material, dilatable under the effect of fluid pressure, designed to be inserted under the tissue and/or the skin requiring repair, and comprising a flexible connecting line (7) designed to connect said body (2) to a source of dilatation fluid, characterized in that the whole system, made up of said flexible body (2) and said line (7), is inserted in a rigid hollow sheath (10) round-shaped at at least one of its ends (12).

2. A tissue expansion prosthesis according to claim 1, characterized in that said dilatable flexible hollow body (2) and said rigid hollow sheath (10) have an oblong coaxial tubular shape.

3. A tissue expansion prosthesis according to claim 1, characterized in that said flexible tubular body (2) is made of silicone.

4. A tissue expansion prosthesis according to claim 1, characterized in that said dilatation line (7) is made of a flexible biocompatible synthetic rubber tube, sealed to the end (4) of said dilatable tubular body (2), opposite said round-shaped end (12) of said rigid sheath (10).

5. A tissue expansion prosthesis according to claim 1, characterized in that said dilatable tubular body (2) presents one side with excess thickness (5) providing a flat pad running lengthwise from end to end.

6. A tissue expansion prosthesis according to one of claims 1 to 5, characterized in that said round-shaped end (12) of said rigid sheath (10) comprises a flexible wire (14) having one end attached to said round-shaped end (13), and its other end attached to a rigid rod (15), this system being in turn protected by a cap (16) encasing the whole of said round-shaped end (12).

7. A tissue expansion prosthesis according to claim 5, characterized in that said excess thickness (5) providing a flat pad is extended at one end (4) of said hollow body (2), such extension (20) being fitted with a valve (21) connected via said connecting line (7) to said dilatable hollow body (2).

FIG.1

FIG.2

FIG.3

FIG.4

FIG.5

FIG.6